# EUROPÄISCHE PATENTANMELDUNG

(11) **EP 3 461 545 A1**
(43) Veröffentlichungstag der Anmeldung: **03.04.2019**
(21) Anmeldenummer: 17194270.9
(22) Anmeldetag: 30.09.2017
(51) Int. Cl.: B01D 3/12, B01D 15/18, C07D 311/80, B01D 11/02

(54) **KURZWEGDESTILLATION IM VAKUUM ZUR ANREICHERUNG VON NATURSTOFFEN**

(71) Anmelder: Bionorica Ethics GmbH, 92318 Neumarkt (DE)
(72) Erfinder: RUTZ, Andreas, 91741 Dornhausen (DE); ENGLERT, Michael, 90478 Nürnberg (DE)
(74) Vertreter: Simandi, Claus

(57) **Zusammenfassung**

Die vorliegende Erfindung betrifft ein Verfahren zur Herstellung, Gewinnung und Anreicherung von Dronabinol (Δ9-THC) als auch von Naturstoffen aus Pflanzenextrakten.

## Beschreibung

Die vorliegende Erfindung betrifft ein Verfahren zur Herstellung, Gewinnung und Anreicherung von Dronabinol (Δ9-THC) als auch von Naturstoffen aus Pflanzenextrakten.

Cannabis (Hanf) gehört zusammen mit der Gattung Humulus (Hopfen) der Familie der Cannabaceae an, wobei jedoch Humulus keine Cannabinoide enthält. Innerhalb der Gattung Cannabis erfolgt eine botanische und chemotaxonomische Differenzierung und zwar in den Arten Cannabis sativa Linnaeus, Cannabis indica LAM und Cannabis ruderalis oder in die "Sammelart" Cannabis sativa L., bestehend aus den Unterarten Cannabis sativa ssp. sativa und ssp. indica. Darüber hinaus wird Cannabis in einen Drogen- und Faserhanf unterschieden, wobei die Unterscheidung aufgrund des mengenmäßigen Verhältnisses der Hauptcannabinoide Cannabidiol (CBD) und Δ⁹-Tetrahydrocannabinol (Δ⁹-THC) erfolgt (INN: Dronabinol). Faserhanf (auch: Nutzhanf, industrial hemp) wird hauptsächlich zur industriellen Fasergewinnung herangezogen und darf maximal einen Δ⁹-THC-Gehalt von 0,2 % aufweisen (z.B. Deutschland u.a.), während der Drogentyp einen Δ⁹-THC-Gehalt von ca. 5-35 % aufweisen kann (Marihuana, Haschisch). Cannabis sativa L. enthält über 400 verschiedene Inhaltsstoffe, davon gehören mehr als 60 Verbindungen der Klasse der Cannabinoide an. Die wichtigsten Cannabinoide sind im Folgenden dargestellt:
*Cannabigerol-artige (CBG) :* Cannabigerol ((E)-CBG-C₅), Cannabigerol Monomethylether ((E)-CBGM-C₅ A), Cannabinerolsäure A ((Z)-CBGA-C₅ A), Cannabigerovarin ((E)-CBGV-C₃), Cannabigerolsäure A ((E)-CBGA-C₅ A), Cannabigerolsäure A Monomethylether ((E)-CBGAM-C₅ A), Cannabigerovarinsäure A ((E)-CBGVA-C₃ A) ;
*Cannabichromen-artige (CBC) :* Cannabichromen (CBC-C₅), Cannabichromensäure A (CBCA-C₅ A), Cannabichromevarin (CBCV-C₃), Cannabichromevarinsäure A (CBCVA-C3 A);
*Cannabidiol-artige (CBD):* Cannabidiol (CBD-C₅), Cannabidiol Monomethylether (CBDM-C₅), Cannabidiol-C4 (CBD-C₄), Cannabidivarin (CBDV-C₃), Cannabidiorcol (CBD-C₁), Cannabidiolsäure (CBDA-C₅), Cannabidivarinsäure (CBDVA-C₃) ;
*Cannabinodiol-artige (CBND) :* Cannabinodiol (CBND-C₅), Cannabinodivarin (CBND-C₃);
*Tetrahydrocannabinol-artige (THC):* Δ9-Tetrahydrocannabinol (Δ9-THC-C₅), Δ9-Tetrahydrocannabinol-C4 (Δ9-THC-C₄), Δ9-Tetrahydrocannabivarin (Δ9-THCV-C₃), Δ9-Tetrahydrocannabiorcol (Δ9-THCO-C₁), Δ9-Tetrahydrocannabinolsäure (Δ9-THCA-C₅ A), Δ9-Tetrahydrocannabinolsäure B (Δ9-THCA-C₅ B), Δ9-Tetrahydrocannabinolsäure-C4 (Δ9-THCA-C₄ A und/oder B), Δ9-Tetrahydrocannabivarinsäure A (Δ9-THCVA-C₃ A), Δ9-Tetrahydrocannabiorcolsäure (Δ9-THCOA-C₁ A und/oder B), (-)-Δ8-trans-(6aR,10aR)-Δ8-Tetrahydrocannabinol (Δ8-THC-C₅), (-)-Δ8-trans-(6aR,10aR)-Tetrahydrocannabinolsäure A (Δ8-THCA-C₅ A); (-)-(6aS,10aR)-Δ9-Tetrahydrocannabinol ((-)-cis-Δ9-THC-C₅);
*Cannabinol-artige (CBN):* Cannabinol CBN-C₅, Cannabinol-C4 (CBN-C₄), Cannabivarin (CBN-C₃), Cannabinol-C2 (CBN-C₂), Cannabiorcol (CBN-C₁), Cannabinolsäure A (CBNA-C₅ A), Cannabinolmethylether (CBNM-C₅)
*Cannabitriol-artige (CBT):* (-)-(9R,10R)-trans-Cannabitriol ((-)-trans-CBT-C₅), (+)-(9S,10S)-Cannabitriol ((+)-trans-CBT-C₅), (±)-(9R,10S/9S,10R)-Cannabitriol ((±)-cis-CBT-C₅), (-)-(9R,10R)-trans[10-O-Ethyl-cannabitriol] ((-)-trans-CBT-OEt-C₅), (±)-(9R,10R/9S,10S)-Cannabitriol-C₃ ((±)-trans-CBT-C₃),8,9-Dihydroxy-Δ6a(10a) tetrahydrocannabinol (8,9-Di-OH-CBT-C₅), Cannabidiolsäure A (CBDA-C₅ 9-OH-CBT-C5 ester), (-)-(6aR,9S,10S,10aR)-9,10-Dihydroxy-hexahydrocannabinol, Cannabiripsol Cannabiripsol-C5, (-)-6a,7,10a-Trihydroxy-Δ9-tetrahydrocannabinol ((-)-Cannabitetrol), 10-Oxo-Δ6a(10a) tetrahydrocannabinol (OTHC);
*Cannabielsoin-artige (CBE)* : (5aS,6S,9R,9aR)- C₅-Cannabielsoin (CBE-C₅), (5aS,6S,9R,9aR)-C₃-Cannabielsoin (CBE-C₃), (5aS,6S,9R,9aR)-Cannabielsoinsäure A (CBEA-C₅ A), (5aS,6S,9R,9aR)-Cannabielsoinsäure B (CBEA-C₅ B), (5aS,6S,9R,9aR)-C3-Cannabielsoinsäure B (CBEA-C₃ B), Cannabiglendol-C3 (OH-iso-HHCV-C₃), Dehydrocannabifuran (DCBF-C₅), Cannabifuran (CBF-C₅);
*Isocannabinoide:* (-)-Δ7-trans-(1R,3R,6R)-Isotetrahydrocannabinol, (±)-Δ7-1,2-cis-(1R,3R,6S/1S,3S,6R)-Isotetrahydrocannabivarin, (-)-Δ7-trans-(1R,3R,6R)-Isotetrahydrocannabivarin;
*Cannabicyclol-artige (CBL):* (±)-(1aS,3aR,8bR,8cR)-Cannabicyclol (CBL-C₅), (±)-(1aS,3aR,8bR,8cR)-Cannabicyclolsäure A (CBLA-C₅ A), (±)-(1aS,3aR,8bR,8cR)-Cannabicyclovarin (CBLV-C₃);
*Cannabicitran-artige (CBT):* Cannabicitran (CBT-C₅);
*Cannabichromanon-artige (CBCN) :* Cannabichromanon (CBCN-C₅), Cannabichromanon-C3 (CBCN-C₃), Cannabicoumaronon (CBCON-C₅).

Neben den o. g. erwähnten Cannabinoiden finden sich deren zugehörige Carbonsäuren in der Rohdroge. Diese Carbonsäuren sind biosynthetische Precursors.

Cannabiszubereitungen üben eine Vielzahl therapeutischer Wirkungen aus, darunter antispastische, analgetische, antiemetische, neuroprotektive, anti-inflammatorische sowie Wirkungen bei psychiatrischen Erkrankungen (Grotenhermen F, Müller-Vahl K: The therapeutic potential of cannabis and cannabinoids. Dtsch Arztebl Int 2012; 109(29-30): 495-501. DOI: 10.3238/arztebl.2012.0495).

In Deutschland ist seit 2011 ein Cannabisextrakt, der THC (Dronabinol) und CBD im Verhältnis 1:1 enthält (Nabiximols), für die Behandlung der mittelschweren bis schweren, therapieresistenten Spastik bei Multipler Sklerose (MS) als Sublingualspray (Sativex) arzneimittelrechtlich zugelassen.

Im Folgenden sind die chemischen Strukturen einiger Cannabinoid-Wirkstoffe und die Nomenklatur der beiden Wirkstoffe des Tetrahydrocannabinols, deren IUPAC Namen (6aR-trans)-6a,7,8,10a-tetrahydro-6,6,9-trimethyl-3- pentyl-6H-dibenzo[b,d]pyran-1-ol oder Δ9-THC und (6aR-trans)-6a,7,10,10a- tetrahydro-6,6,9-trimethyl-3-pentyl-6H-dibenzo[b,d]pyran-1-ol oder Δ8-THC sind, angegeben.

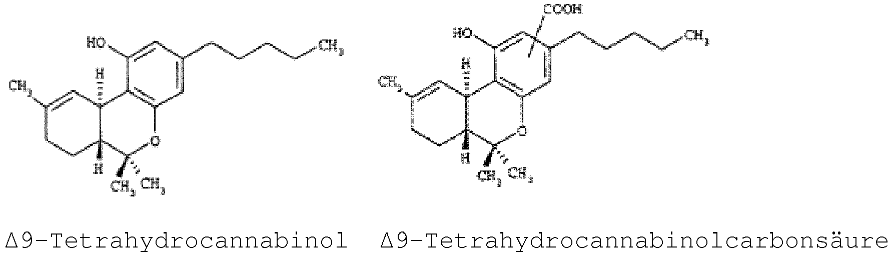

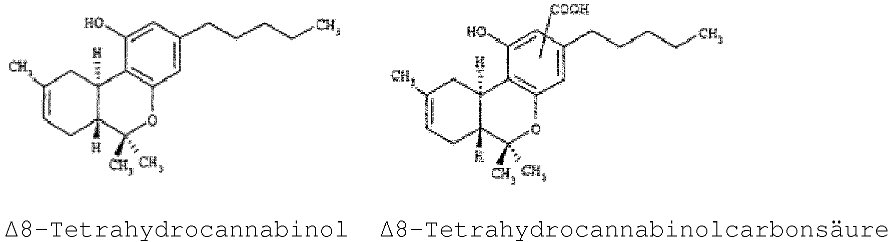

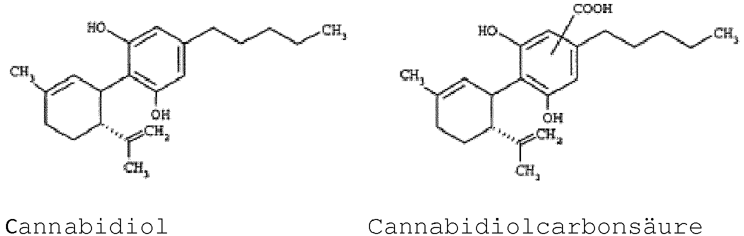

Für die Zwecke der vorliegenden Erfindung soll der Begriff "Tetrahydrocannabinol" oder "THC" - sofern nichts anderes bezeichnet ist - sämtliche Isomere, insbesondere Doppelbindungsisomere, umfassen.

THC kann synthetisch hergestellt werden (EP2314580B1).

DE 100 51 427 C1 (Müller) beschreibt einen THC-haltigen Primärextrakt mittels CO₂-Extraktion aus Cannabis / Hanf unter Anwendung über- oder unterkritischem Druck und Temperaturbedingungen. Hierzu wird eine SFC- oder SFE-Anlage verwendet ("Supercriticalfluid-chromatography").

Eine geeignete Kurzwegdestillation im Vakuum zur Extraktion von THC in hoher Reinheit aus einem Cannabisrohmaterial wird jedoch im Stand der Technik nicht beschrieben. Im Stand der Technik wird die einfache Vakuumdestillation von THC offenbart (WO 00/25127A1) und EP1051084B1 beschreibt z.B. eine Wasserdampfdestillaton aus Hanf.

WO2017055619A1 beschreibt eine Kurzwegdestillation für Cannabidiol.

Daher besteht die Aufgabe eine verbesserte Destillation zur Extraktion von THC bereitzustellen, so dass möglichst ein THCangereicherter Extrakt, gar THC in hoher Reinheit erhalten wird.

Die Aufgabe wird durch die Patentansprüche gelöst.

Daher betrifft die Erfindung ein Verfahren zur Extraktion von THC aus Cannabis-Pflanzenmaterial, wobei eine Kurzwegdestillation im Vakuum durchgeführt wird (nachstehend: erfindungsgemäßes Verfahren).

Im Sinne dieser Erfindung bedeutet Destillation ein thermisches Trennverfahren, um verdampfbare Flüssigkeiten aus einer Gasphase zu gewinnen und von schwer verdampfbaren Stoffen abzutrennen, wobei ein THC-haltiger Extrakt im Destillat angereichert wird. "Vakuumdestillation" im Sinne dieser Erfindung bedeutet, dass die Destillation im Vakuum bei 0,001 bis 50 mbar, vorzugsweise 0,001 bis 10 mbar, besonders bevorzugt 0,001 bis 1 mbar im so genannten Feinvakuumbereich erfolgt.

Weiterhin ist bevorzugt, dass die Verdampfertemperatur 120 bis 240 Grad Celsius beträgt, insbesondere 150 bis 230 Grad Celsius beträgt.

Wesentlich ist, dass das erfindungsgemäße Verfahren zur Vakuumdestillation mittels einer Kurzwegdestillation im Vakuum erfolgt. "Kurzwegdestillation im Vakuum" im Sinne dieser Erfindung bedeutet, dass die Gasphase im angelegten Feinvakuum nur einen sehr kurzen Weg von vorzugsweise 10 cm, insbesondere 5 cm, insbesondere 2 cm zwischen der Verdampferwand und dem Kondensator zurückzulegen hat. Beispielsweise kann ein Kurzwegverdampfer verwendet werden, der konstruktiv einem konventionellen Dünnschichtverdampfer entspricht, wobei jedoch der Kondensator in das Innere des Verdampferzylinders integriert ist, so dass der Weg den die Dämpfe bis zum Kondensator zurücklegen müssen, sehr kurz ist und Drücke von 0,001 mbar erreicht werden können.

Ein geeigneter Dünnschichtverdampfer (engl.: thin film evaporator) im Sinne dieser Erfindung weist eine im Wesentlichen zylindrische, mit Dampf beheizte Innenwandung auf, auf der mittels rotierender Verteilorgane eine dünne Schicht eines Primärextrakts aufgebracht wird. Die motorisch angetriebenen Verteilorgane werden benötigt, um das rasch auf den Platten verdampfende Gemisch auszubringen und zu verteilen.

In einer weiteren Ausführungsform beschleunigt ein Wischersystem den Verdampfungsprozess, indem es die dünne Schicht (Produktfilm) turbulent hält und den Wärmeübergang und Stoffaustausch optimiert. Die leichter siedenden Fraktionen der eingeförderten Rohware bzw. Rohextrakt verdampfen innerhalb kurzer Zeit direkt aus dem Produktfilm. Die benötigte Verweilzeit des Produktes auf der beheizten Verdampferinnenwand ist dadurch minimal. Dank kurzer Verweilzeit, niedriger Verdampfungstemperatur und der sofortigen anschließenden Abkühlung des Konzentrats wird die thermische Schädigung und Belastung der Cannabinoide, insbesondere THC minimiert. Besonders vorteilhaft ist, dass der eingebrachte Rohextrakt ohne weiteres Lösungsmittel aufgebracht werden kann.

Erfindungsgemäß bevorzugt ist jedoch ein Kurzwegverdampfer oder ein entsprechend adaptierter Dünnschichtverdampfer in einer Vakuumvorrichtung.

Weiterhin ist bevorzugt, dass das erfindungsgemäße Verfahren zur Kurzwegdestillation im Vakuum mit mindestens einer zusätzlichen Kolonne bzw. Trennkolonne durchgeführt wird. Eine erfindungsgemäße geeignete Kolonne ist z.B. eine Kolonne DN 60 (ø50mm, L=360 cm) mit Vakuummantel und TI-Stutzen aus Borosilikat oder Edelstahl. Die Kolonne kann übliche Böden als auch Füllkörper oder Kompartimente aufweisen, so dass vorzugsweise mindestens 10 Trennstufen erreicht werden. Solche erfindungsgemäßen Trennkolonnen sind z.B. bei VTA Verfahrenstechnische Anlagen GmbH & Co. KG, Niederwinkling (DE) oder UIC GmbH, Alzenau-Hörstein erhältlich. In einer weiteren Ausführungsform beträgt die Länge der Kolonne mindestens 2,50 m, vorzugswiese 2,70 m oder mehr als 3 m.

In einer weiteren bevorzugten Ausführungsform der Erfindung kann die Kurzwegdestillation im Vakuum mit einer zusätzlichen Kolonnendestillation ausgestattet sein. Insbesondere kann ein Kurzwegverdampfer, entsprechend adaptierter Dünnschichtverdampfer mit einer Trennkolonne entsprechend bestückt werden. Im Sinne dieser Erfindung ist eine Kolonnendestillation ebenfalls eine geeignete Rektifikation im Vakuum, auch unter Rückfluss, welche vorzugsweise 10 Trennstufen aufweist. Die Rektifikation erlaubt die sichere Abtrennung von CBD und Anreicherung von THC auf mehr als 80 %.

Bevorzugt ist weiterhin, dass die erfindungsgemäße Kurzwegdestillation im Vakuum mit einer gekoppelten oder verbundenen Kolonnendestillation bzw. gekoppelten oder verbundenen Trennkolonne bei einem Druck von 0,001 bis 50 mbar, vorzugsweise 0,001 bis 10 mbar, insbesondere 0,001 bis 1 mbar erfolgt. Weiterhin ist bevorzugt, dass die Temperatur 120 bis 240 Grad Celsius beträgt, insbesondere 150 bis 230 Grad Celsius beträgt. In einer bevorzugten Ausführungsform beträgt der Druck bis 5 - 10 mbar und die Temperatur 200 bis 230 Grad Celsius. Dies erlaubt die sichere THC Anreicherung, wobei andere Cannabinoide vollständig abgereichert werden.

Besonders bevorzugt ist weiterhin, dass die erfindungsgemäße Kurzwegdestillation im Vakuum mit einem "Centrifugal Partition Chromatography (CPC)" Verfahren kombiniert wird und zwar in Reihe vor oder nachgeschaltet. Insbesondere kann die Kurzwegdestillation im Vakuum mit einem CPC-Verfahren gekoppelt oder verbunden sein.

Daher betrifft die Erfindung in einer weiteren Aufgabe ein Verfahren zur Trennung und/oder Reinigung von Naturstoffen aus Pflanzenextrakten, insbesondere Cannabinoiden aus einem Cannabisextrakt.

Zur Lösung dieser Aufgabe wird ein Verfahren zur Trennung und/oder Reinigung von Naturstoffen aus Pflanzenextrakten, insbesondere von Cannabinoiden aus einem Cannabisextrakt durchgeführt, enthaltend mindestens einen Flüssig-flüssig-Verteilungschromatographieschritt, wobei ein Lösungsmittel durch Zentrifugalkraft stationär gehalten wird mit einer zweiten nicht mischbaren flüssigen Phase in der mobilen Phase und eine Kurzwegdestillation im Vakuum vor- oder nachgeschaltet wird, wobei eine oder mehrere Fraktionen oder ein oder mehrere Reinstoffe abgetrennt wird/werden.

Das CPC-Verfahren kann zur Gewinnung und Anreicherung von Pflanzeninhaltsstoffen aus Pflanzenextrakten im analytischen, semipräparativen sowie präparativen Maßstab eingesetzt werden. Die CPC ist ein flüssig-flüssig Chromatographieverfahren unter Verwendung eines zumeist zweiphasigen Lösungsmittelsystems.

Das CPC Verfahren ermöglicht eine nahezu verlustfreie Separation hochkomplexer Substanzgemische aus Rohextrakten. Hersteller solcher Zentrifugal-Verteilung-Chromatographen zur Durchführung einer CPC ist z.B. Kromaton S.a.r.l (Annonay, FR) oder Armen Instrument Sas (Saint-Avé, FR).

Bei dem CPC Verfahren wird, wie bei gängigen flüssig-flüssigchromatographischen Methoden, wie zum Beispiel der High Speed Countercurrent Chromatography (HSCCC), ein 2-phasiges Lösungsmittelgemisch eingesetzt. Es kann wahlweise die obere oder die untere Phase als stationäre Phase verwendet werden. Anders jedoch als bei der HSCCC wird bei der CPC nicht mit einer Kapillarspule, sondern mit einem mit mehreren hundert Trennkammern versehenen Rotor gearbeitet. In diesen direkt hintereinander geschalteten Kammern findet die Verteilung der im Pflanzenextrakt enthaltenen Substanzen zwischen mobiler und stationärer Phase statt.

Das System wird während der Trennung in schnelle Rotation versetzt (bis zu 2.500 rpm). Dadurch wird zum einen, je nach Durchflußrichtung, die gewünschte Phase im Rotor der CPC zurückgehalten und zum anderen die Trennung der beiden Phasen durch die Zentrifugalkraft beschleunigt. Dies ermöglicht die Verwendung großer Flußgeschwindigkeiten und folglich den Durchsatz großer Substanzmengen in kurzer Zeit, so dass eine präparative Anwendung dieser Trenntechnik möglich ist.

Besonders bevorzugt ist weiterhin, dass die erfindungsgemäße Kurzwegdestillation im Vakuum mit einem "Sequential Centrifugal Partition Chromatography (sCPC)"-Verfahren kombiniert wird und zwar in Reihe vor oder nachgeschaltet. Insbesondere kann die Kurzwegdestillation im Vakuum mit einem sCPC-Verfahren gekoppelt oder verbunden sein.

Zur Lösung dieser oben genannten Aufgabe wird ein Verfahren zur Trennung und/oder Reinigung von Naturstoffen aus Pflanzenextrakten, insbesondere von Cannabinoiden aus einem Cannabisextrakt durchgeführt, enthaltend mindestens einen Flüssig-flüssig-Verteilungschromatographieschritt, wobei ein kontinuierlicher Wechsel der stationären Phase zur mobilen Phase und umgekehrt erfolgt und eine Kurzwegdestillation im Vakuum vor- oder nachgeschaltet wird, wobei eine oder mehrere Fraktionen oder ein oder mehrere Reinstoffe abgetrennt wird/werden.

Charakteristisch für sCPC ist der kontinuierliche Wechsel der stationären Phase zur mobilen Phase und umgekehrt, d.h. es kann die dichtere oder weniger dichte Phase als mobile Phase ausgewählt werden und ein Wechsel dieser Auswahl ist während einer Trennung möglich. Die zu trennenden Stoffe werden in einer Flüssig-Flüssig-Zentrifugalsäule aufgrund eines unterschiedlichen Verteilungskoeffizienten mit verschiedenen Geschwindigkeiten in den beiden Phasen verschiedener Dichte bewegt.

Eine sCPC-Apparatur weist mindestens einen Rotor mit vielen runden Metallplatten auf in denen sich beispielsweise über Eintausend in Serie verbundene Trennkammern (auch Rotorkammern genannt unter Ausbildung einer rotierenden Trennsäule) befinden. Mit Hilfe einer Pumpe wird im Rotor die stationäre flüssige Phase von einer mobilen flüssigen Phase (kontinuierlich) durchströmt. Der Rotor wird z.B. auf ca. 1.000 U/min und mehr beschleunigt, so dass die Zentrifugalkraft die Trennung der beiden flüssigen Phasen aufgrund der Dichteunterschiede in den einzelnen Kammern bewirkt. Sobald die flüssige mobile Phase im Gleichgewicht (Equilibrium) mit der flüssigen stationären Phase liegt, kann die Probe bzw. das Substanzgemisch in den Rotor injiziert werden.

Die Stofftrennung erfolgt aufgrund verschiedener Adsorption in der mobilen bzw. stationären Phase oder des jeweiligen Verteilungskoeffizienten (K) eines Stoffes zur mobilen/stationären Phase, wobei die Stoffe durch die einzelnen Trennkammern zum Rotorausgang bewegt und fraktioniert werden. Der charakteristische alternierende Wechsel von stationärer Phase und mobiler Phase erfolgt durch Steuerung der eingesetzten Pumpen mittels Ventilen in einer zu wählenden Frequenz. In einer zu wählenden Zeitdauer ("Duration of pumping, Pumpdauer") wird zu der jeweils angesteuerten Phase das zugehörige Lösungsmittel eingepumpt und zwar jeweils an den beiden Enden eines Rotors, so dass die Pumpen entgegengesetzt angeordnet sind.

Die Probe bzw. das Substanzgemisch wird vorzugsweise intermediär in den Rotor eingespeist. In einer weiteren Ausführungsform können zwei oder mehrere Rotoren gekoppelt werden.

Die Rotorkammern werden vorzugsweise im Verhältnis 50/50 (Volumen %) mit einer oberen und einer unteren flüssigen Phase befüllt. Das Substanzgemisch wird mittels Pumpen kontinuierlich zwischen die beiden rotierenden Trennsäulen mit einer definierten Flussrate eingespeist und die Trennung erfolgt zyklisch und zeitversetzt, wobei in einem ersten Schritt die obere Phase als mobile Phase dient ("Ascending") und in einem zweiten Schritt die untere Phase als mobile Phase dient ("Descending").

Entsprechend der Konzentration und der unterschiedlichen Verteilungskoeffizienten der Substanzen im Substanzgemisch erfolgt die Auftrennung in zwei Produktströme.

Geeignete Geräte und Apparaturen können von Armen Technologies (Frankreich) unter dem Namen "True Moving Bed CPC" erhalten werden.

Das sCPC-Verfahren kann zur Gewinnung und Anreicherung von Pflanzeninhaltsstoffen aus Pflanzenextrakten im analytischen, semipräparativen sowie präparativen Maßstab eingesetzt werden. Die sCPC ist ein flüssig-flüssig Chromatographieverfahren unter Verwendung eines zumeist zweiphasigen Lösungsmittelsystems.

Nachstehende Gattungen sind für die Pflanzenextrakte erfindungsgemäß bevorzugt:
*Equiseti, Juglandis, Millefolii, Quercus, Taraxaci, Althaeae, Matricariae, Centaurium, Levisticum, Rosmarinus, Angelica, Artemisia, Astragalus, Leonurus, Salvia, Saposhnikovia, Scutellaria, Siegesbeckia, Armoracia, Capsicum, Cistus, Echinacea, Galphimia, Hedera, Melia, Olea, Pelargonium, Phytolacca, Primula, Salix, Thymus, Vitex, Vitis, Rumicis, Verbena, Sambucus, Gentiana, Cannabis, Silybum.*

Nachstehende Arten sind für die Pflanzenextrakte erfindungsgemäß bevorzugt:
*Equiseti herba* (Schachtelhalmkraut), *Juglandis folium* (Walnussblätter), *Millefolii herba* (Schafgarbenkraut), *Quercus cortex* (Eichenrinde), *Taraxaci herba* (Löwenzahnkraut), *Althaeae radix* (Eibischwurzel) und *Matricariae flos* (bzw. *Flos chamomillae* (Kamilleblüten)), *Centaurium erythraea (Tausendgüldenkraut), Levisticum officinale (Liebstöckel), Rosmarinus officinalis (Rosmarin), Angelica dahurica (Sibirische Engelwurz, PinYin Name: Baizhi), Angelica sinensis (Chinesische Angelika, PinYin Name: Danggui), Artemisia scoparia (Besen-Beifuß, PinYin Name: Yinchen), Astragalus membranaceus (var. Mongolicus) (Traganthwurzel, Chin.: Huang-Qi), Leonurus japonicus (Löwenohr, Chin.: T'uei)*, *Salvia miltiorrhiza (Rotwurzel-Salbei, Chin.: Danshen), Saposhnikovia divaricata (Siler, PinYin Name: Fangfeng), Scutellaria baicalensis (Baikal-Helmkraut, Banzhilian), Siegesbeckia pubescens (Himmlisches Kraut, PinYin Name: Xixianciao), Armoracia rusticana (Meerettich), Capsicum sp. (Paprika), Cistus incanus (Zistrose), Echinacea angustifolia (Sonnenhut), Echinacea purpurea (Sonnenhut), Galphimia glauca, Hedera helix (Efeu), Melia toosendan (Chinesische Holunderfrüchte, Chin.: Chuan Lian Zi), Olea europaea (Olive), Pelargonium sp. (Pelagornien), Phytolacca americana (Kermesbeeren), Primula veris (Schlüsselblume), Salix sp. (Weide), Thymus L. (Thymian), Vitex agnus castus (Mönchspfeffer), Vitis vinifera (Edle Weinrebe),* Rumicis herba (Sorrel herb), *Verbena officinalis* (Eisenkraut), *Sambucus nigra* (Schwarzer Holunder), *Gentiana lutea* (Gelber Enzian), *Cannabis sativa* (Hanf), *Silybum marianum* (Mariendistel).

Ebenfalls sind Mischungen der vorstehenden Gattungen und / oder Arten erfindungsgemäß umfasst.

Vorstehende Arten und Gattungen sind besonders reich an heilbringenden Naturstoffen und sind als Arzneimittelpflanzen beschrieben, wie z.B. in den Produkten auf der Basis von Pflanzenextrakten der Bionorica SE (z.B. Bronchipret®, Imupret®, Sinupret®). Ferner weisen solche Pflanzen übliche charakteristische Stoffklassen auf, wie Flavonoide, Polyphenole, etc.

In einer bevorzugten Ausführungsform wird der Pflanzenextrakt aus einem ersten Lösungsmittel, wie Alkohole, Ethanol, Wasser, Kohlenwasserstoffe, Heptan oder Mischungen davon gewonnen, und die löslichen Anteile eingesetzt.

Beispielsweise können in den Fraktionen solche Stoffklassen von Pflanzenextrakten, wie Alkaloide, Bitterstoffe, Anthocyanine, Anthrachinone, Coumarine, Flavonoide, Glucosinolate, Lactone, Lignane, Lipide, Cannabinoide, Phenole, Polyphenole, Saponine, Terpene, Xanthone in den vorgenannten Verfahren an- oder abgereichert werden, die abgetrennt werden können.

Besonders vorteilhaft können solche Fraktionen und Reinstoffe in hoher Reinheit und Ausbeute in den vorgenannten Verfahren erhalten werden.

Zum Einsatz kommende Lösungsmittel können bei der Flüssig-Flüssig Verteilungschromatographie beispielsweise Skalicka-Woźniak K, Garrard I, A comprehensive classification of solvent systems used for natural product purifications in countercurrent and centrifugal partition chromatography, Nat Prod Rep. 2015 Nov;32(11):1556-61 entnommen werden.

Erfindungsgemäße Beispiele für geeignete Lösungsmittel aus denen zweiphasige Lösungsmittelsysteme (mobile Phase / stationäre Phase) für Pflanzenextrakte bereitgestellt werden können, sind:
a.) Kohlenwasserstoffe wie n-Hexan, Cyclohexan, Isohexan, Heptan, Isooctan;
b.) Ether wie t-butylmethylether, Petrolether, Diethylether;
c.) Halogenierte Lösungsmittel wie Chloroform, Dichlormethan, Benzotrifluorid, Dichlorethan, Tetrachlormethan, Trichlorethan;
d.) Wasser-Lösliche Alkhole wie Butanol, Methanol, Ethanol, Isopropanol;
e.) Wasser-Lösliche Ester wie Ethylacetat, Isopropylacetat;
f.) Acetonitril, Toluol.

Aus diesen vorgenannten Lösungsmitteln lassen sich geeignete zweiphasige Lösungsmittelsysteme herstellen, vorzugsweise solche wie:
n-Heptan/Acetonitril
n-Heptan/Ethylacetat/Acetonitril;
n-Heptan/Ethylacetat/t-Butylmethylether/ Acetonitril;
n-Heptan/Ethylacetat/Methanol/Wasser;
n-Heptan/Ethanol/Wasser.

Erfindungsgemäß wird daher ein Verfahren zur Trennung und/oder Reinigung von Naturstoffen aus Pflanzenextrakten, insbesondere von Cannabinoiden aus einem Cannabisextrakt, insbesondere von THC aus Cannabis-Pflanzenmaterial bereitgestellt, wobei eine Kurzwegdestillation im Vakuum mit einem CPC-Verfahren und / oder sCPC gekoppelt wird bzw. vor- oder nachgeschaltet wird.

Beispielsweise weist das CPC-Verfahren mindestens einen Flüssig-flüssig-Verteilungschromatographieschritt, bzw. die Verwendung eines Zentrifugal-Verteilung Chromatographen zur Flüssig-flüssig-Verteilungschromatographie zur Extraktion z.B. von THC auf, wobei vorzugsweise ein Lösungsmittel aus Hexan, Cyclohexan, Heptan, n-Heptan, iso-Heptan, Octan, n-Octan, iso-Octan ausgewählt ist, das durch Zentrifugalkraft stationär gehalten wird und eine zweite nicht mischbare flüssige Phase als mobile Phase durchgepumpt werden kann.

Beispielsweise weist das sCPC-Verfahren mindestens einen Flüssig-flüssig-Verteilungschromatographieschritt zur Extraktion z.B. von THC auf, wobei vorzugsweise ein Lösungsmittel aus Hexan, Cyclohexan, Heptan, n-Heptan, iso-Heptan, Octan, n-Octan, iso-Octan ausgewählt ist, wobei ein kontinuierlicher Wechsel der stationären Phase zur mobilen Phase und umgekehrt erfolgt.

Die Dichte als auch die Viskosität von Hexan, Cyclohexan, n-Heptan, iso-Octan sind größer als die Dichte/Viskosität von n-Hexan, so dass z.B. gegenüber der zweiten mobilen Phase wie z.B. Acetonitril ein stabileres Zweiphasensystem hergestellt wird, so dass eine bessere Retention der stationären Phase ermöglicht und folglich eine erhöhte Trennleistung erreicht wird; mit z.B. Reinheit für THC von über 95 %, gar 99 %.

In einer weiteren Ausführungsform kann eine oder mehrere Kurzwegdestillationen im Vakuum vor- oder nachgeschaltet werden, so dass eine z.B. Reinheit für THC von über 99,0 %, insbesondere größer als 99,7 % erreicht werden kann.

Weiterhin ist bevorzugt, dass die Kurzwegdestillation im Vakuum an einem ersten Primärextrakt mit mindestens 15 Gew. % an THC oder gewünschtem Cannabinoid erfolgt. In einem ersten Schritt wird daher das Cannabis-Pflanzenmaterial geschnitten, zerkleinert und einer ersten Extraktion unterzogen, beispielsweise eine CO2 Extraktion, wie in DE 100 51 427 C1 beschrieben. Ebenfalls kann alternativ eine Extraktion mit einem Alkan, Propan, Butan, Pentan, Hexan, Heptan, Alkohol, Methanol, Ethanol, Propanol, Isopropanol, Wasser und weiteren flüssigen und gasförmigen Lösungsmittel oder eine Säulenchromatographie erfolgen und auf diese Weise ein Primärextrakt vorzugsweise mit mindestens 15 Gew. % an THC erhalten werden, welcher zur weiteren Kurzwegdestillation im Vakuum eingesetzt wird (supra). Eine CO₂-Extraktion ist jedoch bevorzugt. Bevorzugtes Cannabis-Pflanzenmaterial ist Drogenhanf (Cannabis Sativa).

Weiterhin können im Primärextrakt die Carbonsäuren der Cannabinoide, insbesondere Tetrahydrocannabinolcarbonsäuren, Cannabidiolcarbonsäure decarboxyliert werden. Die Decarboxylierung erfolgt durch Erhitzen bei 120°C unter Vakuum vorzugsweise von 100-200 mbar. Ebenso ist eine kontinuierliche Decarboxylierung mittels Kurzwegdestillation in der Entgaserstufe möglich bei vorzugsweise 120°C und 1 mbar.

Daher ist Gegenstand der Erfindung ebenfalls ein THC-haltiger Extrakt erhältlich nach einem erfindungsgemäßen Verfahren.

In einer weiteren Ausführungsform gemäß dem erfindungsgemäßen Verfahren konnte ein Extrakt mit folgender Zusammensetzung nach einer Kurzwegdestillation im Vakuum erhalten werden:

| | | |
|---|---|---|
| Delta9-THC | % | 92,04 |
| CBC | | 0,83 |
| CBG | % | 1,51 |
| CBN | % | 0,73 |
| VUX | % | 0,00 |

Daher erlaubt das erfindungsgemäße Verfahren eine Anreicherung von THC von mehr als 90 %, insbesondere 92 % als auch 95 % oder mehr unter vorteilhafter Abreicherung von anderen Cannabinoiden und schwer abtrennbaren Verunreinigungen wie VUX.

Weiterhin beträgt der Leichtsiederanteil bezogen auf THC [%] 5 und der Schwersiederanteil bezogen auf THC [%] 3. D.h. durch das erfindungsgemäße Verfahren konnten vorteilhaft fast alle Substanzen, die vor und nach THC sieden, selektiv abgetrennt werden.

Daher betrifft die Erfindung ebenfalls einen THC-haltigen Extrakt erhältlich nach einem erfindungsgemäßen Verfahren aufweisend einen Schwersiedeanteil von weniger als 53 % bezogen auf THC und einen Leichtsiedeanteil von weniger als 5 % bezogen auf THC.

Weiterhin wurde für das erfindungsgemäße Verfahren eine exemplarische Ausgangszusammensetzung, wie folgt eingesetzt:

| | | |
|---|---|---|
| Delta9-THC | % | 78 |
| CBC | % | 0,69 |
| CBG | % | 1,36 |
| CBN | % | 0, 62 |

Das erfindungsgemäße Verfahren erlaubt besonders vorteilhaft die deutliche Reduktion der Leicht- und Schwersiederanteile gegenüber einem eingesetzten Ausgangsmaterial. Dies führt zu einem Extrakt mit spezifischen Eigenschaften wie eine vorteilhafte rückstandsfreie Verdampfbarkeit, welche für die Inhalation von THC in der medizinischen Anwendung besonders geeignet ist, insbesondere für ein Arzneimittel.

Dieser Extrakt lässt sich besonders vorteilhaft für die CPC oder sCPC einsetzen, da die Beladung um Faktor 5 erhöht werden kann im Vergleich zu einem nicht-destillierten Extrakt.

Die erhaltenen Fraktionen, Reinstoffe, Extrakte können Gegenstand einer Galenik oder einer pharmazeutischen Zusammensetzung sein.

Die galenische Herrichtung einer erfindungsgemäßen pharmazeutischen Zusammensetzung oder Mittel, insbesondere ein Arzneimittel (Medikament), kann in fachüblicher Weise erfolgen. Geeignete feste oder flüssige galenische Zubereitungsformen sind beispielsweise Granulate, Pulver, Dragees, Tabletten, (Mikro-) Kapseln, Hartkapseln, Suppositorien, Sirupe, Säfte, Suspensionen, oder Emulsionen, bei deren Herstellung übliche Hilfsmittel wie Trägerstoffe, Spreng-, Binde-, Überzugs-, Quellungs-, Gleit™ oder Schmiermittel, Geschmacksstoffe, Süßungsmittel und Lösungsvermittler, Verwendung finden. Als Hilfsstoffe seien Magnesium-Stearat, Natriumchlorid, Magnesiumcarbonat, Titandioxid, Lactose, Mannit und andere Zucker, Talcum, Milcheiweiß, Gelatine, Stärke, Zellulose und ihre Derivate, tierische und pflanzliche Öle wie Lebertran, Sonnenblumen-, Erdnuss- oder Sesamöl, Polyethylenglycole und Lösungsmittel, wie etwa steriles Wasser und ein- oder mehrwertige Alkohole, beispielsweise Glycerin, genannt.

Weiterhin können als Hilfs- und Zusatzstoffe weitere Detergentien und Tenside vorgesehen sein, wie beispielsweise nachstehend für eine kosmetische Zusammensetzung ausgeführt.

Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert. Sind die beiden Flüssigkeiten Wasser und Öl und liegen Öltröpfchen fein verteilt in Wasser vor, so handelt es sich um eine Öl-in-Wasser-Emulsion (O/W-Emulsion). Der Grundcharakter einer O/W-Emulsion ist durch das Wasser geprägt. Bei einer Wasser-in-Öl-Emulsion (W/O-Emulsion) handelt es sich um das umgekehrte Prinzip, in dem der Grundcharakter hier durch das Öl bestimmt wird. Weiterhin sind Mischsysteme wie Wasser-in-Öl-in-Wasser-Emulsionen (W/O/W-Emulsion) und Öl-in-Wasser-in-Öl-Emulsionen (O/W/O-Emulsionen) bekannt. Alle genannten Emulsionen sind erfindungsgemäß geeignet.

Zu den erfindungsgemäß geeigneten wasserfreien Systemen gehören reine Ölzubereitungen wie z.B. Hautöle. Ebenfalls einsetzbare Pasten enthaltend die erfindungsgemäße Zubereitung und zeichnen sich dadurch aus, dass sie aus den gleichen oder ähnlichen Bestandteilen wie eine Emulsion bestehen, jedoch im Wesentlichen wasserfrei sind. Im Rahmen der vorliegenden Erfindung werden die Begriffe Ölphase und Lipidphase synonym verwendet. In einer weiteren bevorzugten Ausführungsform kann die erfindungsgemäße Zubereitung als weiteren Bestandteil einen Emulgator beinhalten. In einer ganz bevorzugten Ausführungsform kann dieser Emulgator ein O/W-Emulgator sein.

Emulgatoren können vorteilhaft gewählt werden aus der Gruppe der nichtionischen, anionischen, kationischen oder amphoteren Emulgatoren.

Als nichtionischen Emulgator können verschiedene Emulgatoren aus den Gruppen der Partialfettsäureester, Fettalkohole, Sterole, Polyethylengylcole wie z.B. ethoxylierte Fettsäuren, ethoxylierte Fettalkohole und ethoxylierte Sorbitanester, Zuckeremulgatoren, Polyglycerinemulgatoren oder Silikonemulgatoren Verwendung finden.

Als anionische Emulgatoren können verschiedene Emulgatoren aus den Gruppen der Seifen z. B. Natriumstearat, Fettalkoholsulfate, Mono-, Di- und Trialkylphosphosäureester und deren Ethoxylate, Fettsäure Lactat Ester, Fettsäure Citrat Ester oder Fettsäure Citroglycerinester Verwendung finden.

Als kationische Emulgatoren können beispielsweise quaternäre Ammoniumverbindungen mit einem langkettigen aliphatischen Rest z. B. Distearyldimonium Chloride eingesetzt werden.

Unter den amphoteren Emulgatoren können verschiedene Emulgatoren aus den Gruppen Alkylamininoalkancarbonsäuren, Betaine, Sulfobetaine oder Imidazolinderivate Verwendung finden.

Erfindungsgemäß bevorzugt sind natürlich vorkommende Emulgatoren, zu denen beispielsweise Bienenwachs, Wollwachs, Lecithin und Sterole u.a. gehören die ebenfalls bei der Herstellung einer erfindungsgemäßen Zubereitung eingesetzt werden können. In einer bevorzugten Formulierung der erfindungsgemäßen Zubereitung können O/W-Emulgatoren ausgewählt werden aus der Gruppe der Pflanzenproteinhydrolysate und deren Derivate.

Im Sinne der vorliegenden Erfindung können ferner vorteilhaft Substanzen als Zusatzstoffe enthalten sein, ausgewählt aus der Gruppe der Ester aus gesättigten und/oder ungesättigten, verzweigten und/oder unverzweigten Alkancarbonsäuren und/oder Alkencarbonsäuren mit einer Kettenlänge von 3 - 30 Kohlenstoffatomen und gesättigten und/oder ungesättigten, verzweigten und/oder unverzweigten Alkoholen einer Kettenlänge von 3 - 30 Kohlenstoffatomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esterole können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononyliso-nonanoat, 2-Ethylhexylpalmitat, 2-Ethylhexyllaurat, 2- Hexyldecylstearat, 2-Octyldodecyl-palmitat, Oleyloleat, Oleylerucat, Erucyloleat, Erucylerucat sowie synthetische, halbsynthetische und natürliche Gemische solcher Ester, wie z.B. Jojobaöl.

Ferner kann die Ölphase vorteilhaft gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 - 24 C-Atomen, insbesondere 12 - 18 C-Atomen. Die Fettsäuretriglyceride können beispielsweise vorteilhaft gewählt werden aus der Gruppe der synthetischen, halbsynthetischen und natürlichen Öle.

Insbesondere können in den erfindungsgemäßen Zubereitungen zusätzlich als Hilfs- oder Zusatzstoff Antioxidantien und/oder Radikalfänger zugesetzt werden. Vorteilhaft werden solche Antioxidantien gewählt aus der Gruppe der lipophilen Systeme, beispielsweise: natürliche und synthetische Tocopherole, Nordihydroguajaretsäure, Coniferylbenzoat, Butylhydroxyanisol, Butlyhydroxy-tolul, Gallussäureester und verschiedene antioxidative Pflanzen Extrakte. Unter den hydrophilen Systemen sind besonders vorteilhaft anorganischen Schwefelverbindungen, Natriumhydrogensulfit, Cystein oder Ascorbinsäure zu verwenden.

Nachfolgende Beispiele dienen zur weiteren Erörterung der Erfindung, ohne jedoch die Erfindung auf diese Beispiele zu beschränken.

### Beispiel 1:

### 1.1 Kurzwegdestillation V9001

Als Edukt wurde Cannabisextrakt 50-95% Cannabis Sativa verwendet. Das Edukt wurde zuvor bei 80°C im Ölbad erhitzt und in den beheizten Tropftrichter der Kurzwegdestillation überführt. Die eingesetzte Menge betrug 103,3 g. Die Temperatur im Dosiergefäß lag bei 70°C. Die Drehzahl des Rührers betrug 400 rpm und die Temperatur im Verdampfer lag bei 180°C. Der Extrakt wurde mit einer Geschwindigkeit von 180 ml/h - 200 ml/h eingefördert und der Druck in der gesamten Apparatur lag bei 1,2 * 10-1 mbar. Die Destillation dauerte insgesamt 35 Minuten. Es konnten 79,53 g Destillat und 58,15 g Rückstand gewonnen werden was einem Schnittverhältnis von circa 75/25 entspricht. Außerdem bildete sich während der Destillation an der Kühlfalle ein Destillat. Die Menge an Destillat war circa 3 ml. Um den anfallenden Rückstand in eine fließfähige Form zu bekommen war ein Heißluftföhn nötig. Das Destillat, welches anschließend weiterverwendet wurde hatte eine leuchtend gelbe Farbe.

### 1.2 Kurzwegdestillation V9002

Eingesetzt wurde 137,51 g Cannabisextrakt 50-95% Ch. 0000106433. Die Temperatur im Dosiergefäß lag bei 70°C, im Verdampfer bei 165°C. Die Drehzahl war wie zuvor bei 400 rpm eingestellt. Die gesamte Destillation dauerte circa 45 Minuten. Es konnte 90,4 g Destillat gewonnen werden, wobei 61,31 g Rückstand entstanden. In der Kühlfalle bildete sich ein Destillat von circa 3 ml. Das Schnittverhältnis war bei 65/35. Das Destillat, welches anschließend weiterverwendet wurde hatte eine leuchtend gelbe Färbung.

### 1.3 Herstellung der Stammlösung V9001

Als Ausgangsmaterial für die Stammlösung diente das Destillat aus der Kurzwegdestillation. Es wurde die gleiche Konzentration für die Stammlösung verwendet wie auch an der FCPC Anlage, also 0,1 g/ml. Das Destillat aus V9001 wurde bei 80°C im Wasserbad angelöst, anschließend in einen 1000 ml Rundkolben überführt. Die Einwaage betrug 62,26 g. Der 1000 ml Rundkolben wurde dann nochmals in einem Wasserbad bei 80°C angelöst und mit 622 ml Heptan FCPC versetzt und gelöst.

Die Ergebnisse sind in Tabelle 1 dargestellt.

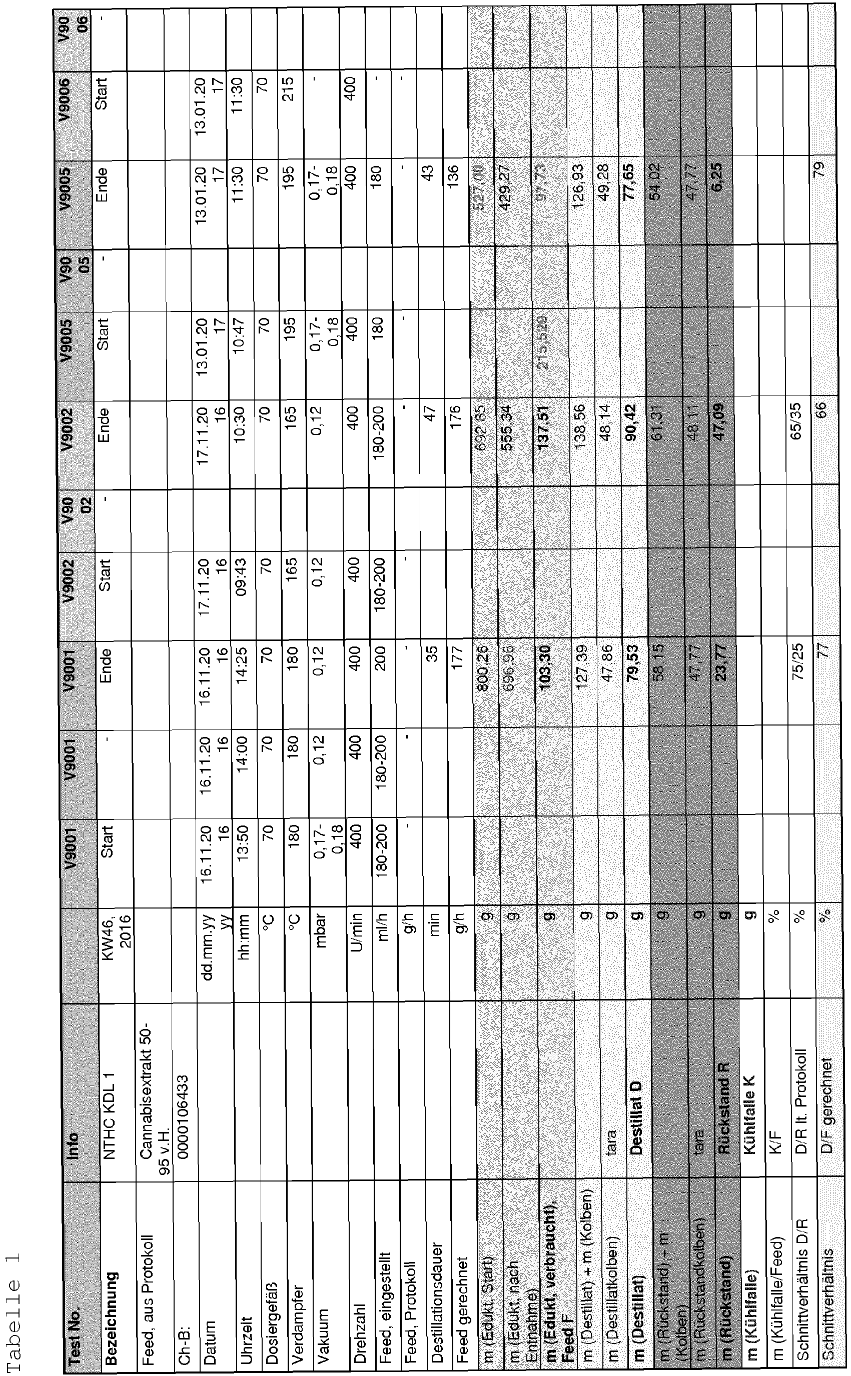

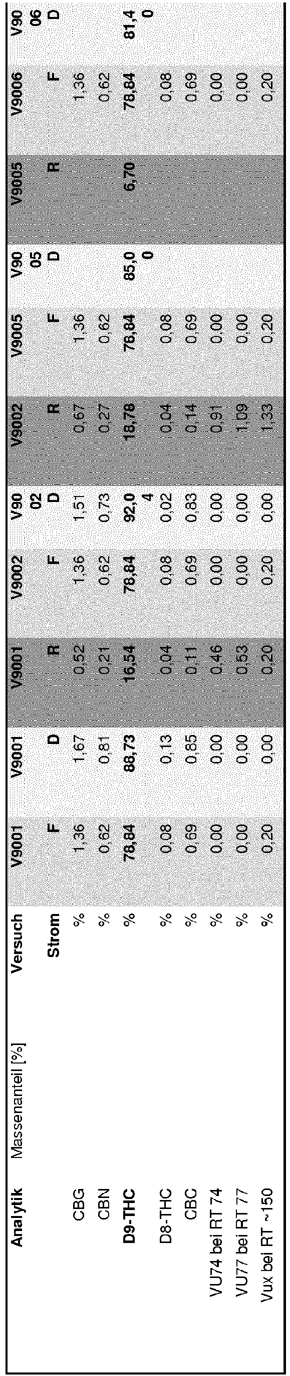

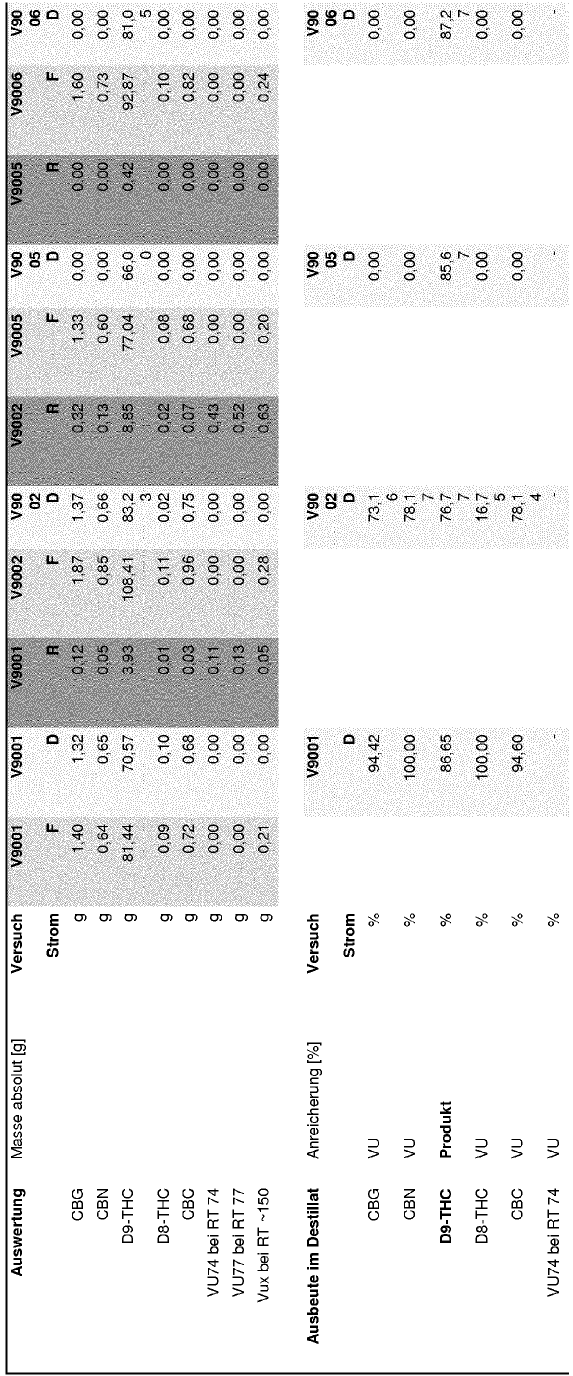

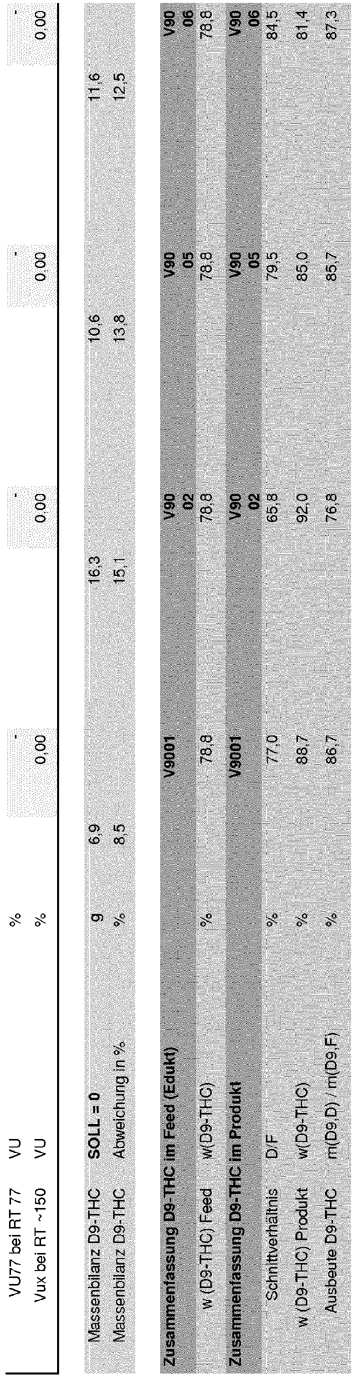

## Patentansprüche

1. Verfahren zur Extraktion von THC aus Cannabis-Pflanzenmaterial, **dadurch gekennzeichnet, dass** eine Kurzwegvakuumdestillation durchgeführt wird.

2. Verfahren zur Extraktion von THC aus Cannabis-Pflanzenmaterial nach Anspruch 1, **dadurch gekennzeichnet, dass** die Kurzwegvakuumdestillation mittels eines Kurzwegverdampfers erfolgt.

3. Verfahren zur Extraktion von THC aus Cannabis-Pflanzenmaterial nach Anspruch 1 oder Anspruch 2, **dadurch gekennzeichnet, dass** die Kurzwegvakuumdestillation mittels einer zusätzlichen Kolonne oder Trennkolonne erfolgt.

4. Verfahren zur Extraktion von THC aus Cannabis-Pflanzenmaterial nach Anspruch 3, **dadurch gekennzeichnet, dass** die Länge der Trennkolonne mindestens 2,50 m beträgt.

5. Verfahren zur Extraktion von THC aus Cannabis-Pflanzenmaterial nach einem der vorstehenden Ansprüche, wobei die Vakuumdestillation an einem ersten Primärextrakt mit mindestens 15 Gew. % an THC erfolgt.

6. Verfahren zur Extraktion von THC aus Cannabis-Pflanzenmaterial nach einem der vorstehenden Ansprüche, wobei der Druck 0,001 bis 50 mbar und die Temperatur 120 bis 240 Grad Celsius beträgt.

7. Verfahren zur Extraktion von THC aus Cannabis-Pflanzenmaterial, **dadurch gekennzeichnet, dass** ein oder mehrere Kurzwegdestillationen im Vakuum mit mindestens einem Flüssig-flüssig-Verteilungschromatographieschritt vor- oder nachgeschaltet wird.

8. Verfahren zur Extraktion von THC aus Cannabis-Pflanzenmaterial, **dadurch gekennzeichnet, dass** ein oder mehrere Kurzwegdestillationen im Vakuum mit mindestens einem Flüssig-flüssig-Verteilungschromatographieschritt vor- oder nachgeschaltet wird, wobei mindestens i.) ein kontinuierlicher Wechsel der stationären Phase zur mobilen Phase und umgekehrt erfolgt und / oder ii.) ein Lösungsmittel durch Zentrifugalkraft stationär gehalten wird mit einer zweiten nicht mischbaren flüssigen Phase in der mobilen Phase.

9. THC-haltiger Extrakt nach einem der Ansprüche 1 bis 8, **dadurch gekennzeichnet, dass** der Extrakt eine rückstandsfreie Verdampfbarkeit aufweist.

10. Arzneimittel, Nahrungsergänzungsmittel enthaltend einen THC-haltigen Extrakt erhältlich nach einem Verfahren gemäß den Ansprüchen 1 bis 8, insbesondere zum Inhalieren.

11. Verfahren zur Trennung und/oder Reinigung von Naturstoffen aus Pflanzenextrakten, insbesondere von Cannabinoiden aus einem Cannabisextrakt, enthaltend mindestens einen Flüssig-flüssig-Verteilungschromatographieschritt, wobei ein Lösungsmittel durch Zentrifugalkraft stationär gehalten wird mit einer zweiten nicht mischbaren flüssigen Phase in der mobilen Phase und eine oder mehrere Kurzwegdestillationen im Vakuum vor- oder nachgeschaltet werden, wobei eine oder mehrere Fraktionen oder ein oder mehrere Reinstoffe abgetrennt wird/werden.

12. Verfahren zur Trennung und/oder Reinigung von Naturstoffen aus Pflanzenextrakten, insbesondere von Cannabinoiden aus einem Cannabisextrakt, enthaltend mindestens einen Flüssig-flüssig-Verteilungschromatographieschritt, wobei ein kontinuierlicher Wechsel der stationären Phase zur mobilen Phase und umgekehrt erfolgt und ein oder mehrere Kurzwegdestillationen im Vakuum vor- oder nachgeschaltet werden, wobei eine oder mehrere Fraktionen oder ein oder mehrere Reinstoffe abgetrennt wird/werden.

13. Verfahren zur Trennung und/oder Reinigung von Naturstoffen aus Pflanzenextrakten nach einem der Patentansprüche 11 und 12, **dadurch gekennzeichnet, dass** die Fraktion(en) solche Stoffklassen von Pflanzenextrakten enthalten ausgewählt aus der Gruppe Alkaloide, Bitterstoffe, Anthocyanine, Anthrachinone, Coumarine, Flavonoide, Glucosinolate, Lactone, Lignane, Lipide, Cannabinoide, Phenole, Polyphenole, Saponine, Terpene, Xanthone.

14. Verfahren zur Trennung und/oder Reinigung von Naturstoffen aus Pflanzenextrakten nach einem der Patentansprüche 11 bis 13, **dadurch gekennzeichnet, dass** mindestens ein Pflanzenextrakt ausgewählt ist aus der Gruppe der Gattungen Equiseti, Juglandis, Millefolii, Quercus, Taraxaci, Althaeae, Matricariae, Centaurium, Levisticum, Rosmarinus, Angelica, Artemisia, Astragalus, Leonurus, Salvia, Saposhnikovia, Scutellaria, Siegesbeckia, Armoracia, Capsicum, Cistus, Echinacea, Galphimia, Hedera, Melia, Olea, Pelargonium, Phytolacca, Primula, Salix, Thymus, Vitex, Vitis, Rumicis, Verbena, Sambucus, Gentiana, Cannabis, Silybum.

15. Verfahren zur Trennung und/oder Reinigung von Naturstoffen aus Pflanzenextrakten nach Anspruch 14, **dadurch gekennzeichnet, dass** mindestens ein Pflanzenextrakt ausgewählt ist aus der Gruppe der Arten Equiseti herba (Schachtelhalmkraut), Juglandis folium (Walnussblätter), Millefolii herba (Schafgarbenkraut), Quercus cortex (Eichenrinde), Taraxaci herba (Löwenzahnkraut), Althaeae radix (Eibischwurzel) und Matricariae flos (bzw. Flos chamomillae (Kamilleblüten)), Centaurium erythraea (Tausendgüldenkraut), Levisticum officinale (Liebstöckel), Rosmarinus officinalis (Rosmarin), Angelica dahurica (Sibirische Engelwurz, PinYin Name: Baizhi), Angelica sinensis (Chinesische Angelika, PinYin Name: Danggui), Artemisia scoparia (Besen-Beifuß, PinYin Name: Yinchen), Astragalus membranaceus (var. Mongolicus) (Traganthwurzel, Chin.: Huang-Qi), Leonurus japonicus (Löwenohr, Chin.: T'uei), Salvia miltiorrhiza (Rotwurzel-Salbei, Chin.: Danshen), Saposhnikovia divaricata (Siler, PinYin Name: Fangfeng), Scutellaria baicalensis (Baikal-Helmkraut, Banzhilian), Siegesbeckia pubescens (Himmlisches Kraut, PinYin Name: Xixianciao), Armoracia rusticana (Meerettich), Capsicum sp.(Paprika), Cistus incanus (Zistrose), Echinacea angustifolia (Sonnenhut), Echinacea purpurea (Sonnenhut), Galphimia glauca, Hedera helix (Efeu), Melia toosendan (Chinesische Holunderfrüchte, Chin.: Chuan Lian Zi), Olea europaea (Olive), Pelargonium sp. (Pelagornien), Phytolacca americana (Kermesbeeren), Primula veris (Schlüsselblume), Salix sp. (Weide), Thymus L. (Thymian), Vitex agnus castus (Mönchspfeffer), Vitis vinifera (Edle Weinrebe), Rumicis herba (Sorrel herb), Verbena officinalis (Eisenkraut), Sambucus nigra (Schwarzer Holunder), Gentiana lutea (Gelber Enzian), Cannabis sativa (Hanf), Silybum marianum (Mariendistel).
